# EUROPEAN PATENT SPECIFICATION

(11) **EP 2 223 677 B1**
(45) Date of publication and mention of the grant of the patent: **22.08.2018**
(21) Application number: 08865045.2
(22) Date of filing: 26.12.2008
(51) Int. Cl.: B29C 43/20, B29L 31/56, B29K 21/00, B29L 31/26, B29K 105/00, B32B 27/08, B29K 27/18, B29C 43/18, B65D 39/00, C09K 3/10, B32B 25/08, B32B 25/14, B32B 27/16, B32B 27/32, B32B 3/30, B32B 3/04

(54) **RUBBER MOLDINGS**
GUMMIFORMTEILE
MOULAGES EN CAOUTCHOUC

(30) Priority: 26.12.2007 JP 2007334011
(43) Date of publication of application: 01.09.2010
(73) Proprietor: Daikyo Seiko, LTD., Tochigi, 327-0813 (JP)
(72) Inventor: KAWACHI, Yasushi, Tochigi, 327-0813 (JP)
(74) Representative: TBK
(86) International application number: PCT/JP2008/073955
(87) International publication number: WO 2009/082034

(56) References cited:
- EP-A2- 0 976 453
- WO-A1-97/05196
- WO-A1-2006/118198
- WO-A1-2007/040261
- JP-B2- 4 064 938
- JP-U- 58 055 052
- US-A- 4 397 903
- US-A- 6 165 402
- US-A1- 2005 001 974

## Description

### Technical Field

The present invention relates to a molded rubber article for sealing a container or an instrument used in medication or medical care application, more specifically a molded rubber article having a fluorine resin film which solidly covers the surface which may contact with chemicals.

### Background Art

As a sealing material for a container or an instrument used in medication and medical care applications, rubber materials are considered to be suitable in view of sealing performance. Examples of sealing materials for a container or an instrument used in medication and medical care applications can be found in documents EP-A-0976453, US-A-4397903 or US-A-6165402. Natural rubbers used to be widely utilized and synthetic rubbers are now widely used for these application. Among other things copolymerized rubbers (IIR) of isobutylene and isoprene are widely used in view of functionality.

For the purpose of preventing cross-linking agents and compounding agents contained in rubbers from eluting into medicinal chemicals stored in a container, preventing contents in a container from absorbing onto bare rubber surface, preventing rubber members from generating fine particles during production process or storage of medicinal chemicals or preventing from aggregation by reaction of silicone oil applied on the bare rubber surface with medicinal solution, laminated rubber plugs are coated by an inactive film which is chemically inactive and has a high heat resistance property, a high chemical resistance property, a high weather resistance property, a high non-viscous property, a high compressive strain resistance property, a high bendability and a low gas and moisture permeation property.

An olefin thermoplastic resin film, e.g. a polypropylene film, a polyethylene and a fluorine resin film are available as such an inactive laminating material, however a fluorine resin film is usually used because it is advantageous in view of a low reactivity to contacting medicinal chemicals, a high water repellency, a high lubricity and a high temperature stability (See patent documents 1 - 6, for example).

Although a fluorine resin film has a high functionality as a medicinal chemical wrapping material because of the high lubricity and a low reactivity, it has a drawback that it is difficult to adhere to a bare rubber surface.

In order to improve the adherence property with a rubber member, it is known to use a medicinal chemical method for applying "a compound liquid of naphthalene and tetrahydrofuran with a dissolved alkali metal like a metallic sodium" or " a liquid ammonia with a dissolved alkali metal" to the surface of a fluorine resin film and an electronic irradiation method for processing the surface of a fluorine resin film by corona discharging or spattering.

Although the adherence property of a fluorine resin film to a bare rubber surface can improve by using such processes, the adhesion strength does not sufficiently improve in a kind of rubber materials and there is a possibility of detachment of a film from the bare rubber surface during use.

For example, a rubber member containing halogen or a rubber member with a high forming temperature (e.g. a halogenated butyl rubber) have a relatively high adherence property to a fluorine resin film, however a regular butyl rubber, a ethylene propylene rubber (EPDM), a styrene elastomer (SEBS, SBS) and a rubber which is composed mostly of polybutadiene or polyisobutylene (including a thermoplastic elastomer) does not have a sufficient adherence property to a fluorine resin film, and there was an interface detachment problem.

It has been considered to use an adhesive agent in order to improve the adherence property.

The workability becomes very poor when a fluorine resin film (having a high chemical resistance property, a high whether resistance property and a high antifouling property) is laminated on the surface of a sheet-shaped member like a wallpaper member because a fluorine resin film is not elastic. Therefore, a technique has been developed for making a wallpaper member with a fluorine resin film on its surface by extruding a fluorine resin layer together with an adhesive agent resin layer (tetrafluoroethylene-hexafluoropyrene-vinylidene fluoride ternary copolymer resin (THV), for example), pressure bonding it to a stretched polyethylene terephthalate (PET) film as an adhesive agent layer, and welding the PET film with a base material sheet (See patent document 7).
Patent document 1: Japanese Laid-open Patent Application No. H2 - 136139
Patent document 2: Japanese Laid-open Patent Application No. 2002 - 177390
Patent document 3: Japanese Laid-open Patent Application No. H7 - 323072
Patent document 4: Japanese Laid-open Patent Application No. H9 - 59396
Patent document 5: Japanese Laid-open Patent Application No. 2002 - 209975
Patent document 6: Japanese Laid-open Patent Application No. 2004 - 216753
Patent document 7: Japanese Laid-open Patent Application No. 2005 - 59409

### Disclosure of Invention

A purpose of the present invention is to provide a molded rubber, as described in claim 1, with a fluorine resin film laminated on the surface, wherein the adhesion strength between the bare rubber surface and the fluorine resin film is improved to prevent detachment of the film from the bare rubber surface.

The inventers of the present invention invented a novel method for enhancing the detachment strength between a fluorine resin film and a bare rubber surface which was weak according to the prior art based on a finding that a laminate structure of a molded rubber article and a fluorine resin film which can prevent interface detachment between layers can be formed by interposing a film having a high adhesion property between a fluorine resin film and a bare rubber surface, heating them under vacuum and forming a two layer laminated structure mold rubber article using mold clamping and venting gas generated during cross-linking reaction, wherein the fluorine resin film, the interposed film and the bare rubber surface are adhesively integrated.

That is to say, a molded rubber article according to the present invention is used to seal a container for storing medicinal chemicals wherein at least the surface of the molded rubber article which may contact with medicinal chemicals is formed by a fluorine resin film layer, and a polyolefin film layer is interposed between the bare rubber surface and the fluorine resin film layer.

It is preferable to use a thermoplastic elastomer as a rubber material and it is more preferable to use at least one selected from a group of an olefin resin, a styrene resin, a vinyl chloride resin, an urethane resin, a polyester resin, a polyamide resin, a fluorine resin, a polybutadiene resin, a polyisobutylene resin, a silicone resin and an ethylene- vinyl acetate resin.

It is preferable to use a polytetrafluoroethylene (PTFE) as the fluorine resin and an ultrahigh molecular weight polyethylene is used as the polyolefin.

It is used a polyethylene of a molecular weight in the range between 1 million and 7 millions as the aforementioned ultrahigh molecular weight polyethylene and it is more preferable to use a polyolefin of a molecular weight of more than 5 millions for enhancing the adhesive integration of the bare rubber surface and the fluorine resin film.

It is preferable to use a fluorine resin film layer of a thickness of 10-200 µm and a polyolefin film layer of a thickness of 10-100µm. The total thickness of the two layers is 20-300µm.

It is preferable to use a molded rubber article according to the present invention as a rubber plug, a syringe gasket, a nozzle cap, a cylinder cap, an inside plug, etc. These molded rubber articles are formed by the following method, for example.

The exemplary method comprises putting a polyolefin film on top of a fluorine resin film on a lower mold tool, putting a sheet-shaped rubber member on top of the polyolefin film, moving down an upper mold tool, mold clamping the stacked sheet/films while vacuuming up the air in a cavity and heating the upper and lower mold tools to a temperature which is higher than the molding temperature of the sheet-shaped rubber member while keeping the mold clamping pressure at 50-200Kg/cm².

### Brief Description of Drawings

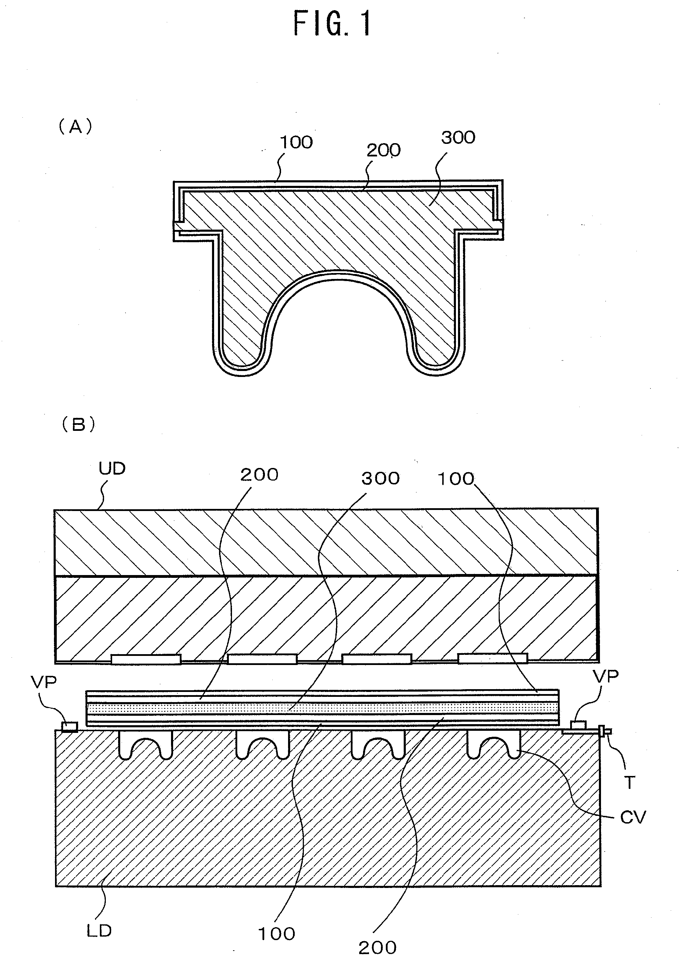

Fig. 1 (A) shows a cross sectional schematic view of a molded rubber article (a rubber plug) according to one embodiment of the present invention. Fig. 1(B) shows a cross sectional schematic view of an exemplary forming process of a rubber plug according to one embodiment of the present invention.

### Explanation of Reference Numerals

UD : upper mold tool, LD : lower mold tool
100: PTFE film
200: ultrahigh molecular weight polyethylene
300: unformed sheet-shaped rubber member
CV : cavity
T : vacuuming up outlet
VP : vacuum packing

### Effect of Invention

A molded rubber article according to the present invention is useful as a sealing member for a container for storing medicinal chemicals like a rubber plug or a cylinder cap because the interface between a bare rubber surface and a polyolefin film layer has a high adherence property, the interface between the polyolefin film layer and a fluorine resin film layer has a high adherence property, and the interfaces are not easily detached.

It is possible to form a molded rubber article according to the present invention as an aforementioned sealing member in an efficient manner by adhesively integrating a bare rubber surface, a polyolefin film and a fluorine resin film and preventing from interface detachment between the layers through a process of laminating these films on a sheet-shaped rubber member and forming in a desired shape.

### Best Mode for Carrying Out the Invention

In the field of sealing materials for medicinal chemical containers, it is prohibited to use materials with an uncertified safety and it is also preferable not to use any adhesive agent.

For medical plastic materials, it is preferable to be nontoxic and hygienic, adaptable to various sterilization methods, e.g. a heat sterilization method, an ethylene oxide sterilization method, an autoclaving method, a radiation method and have a property of high light resistance, high weather resistance, high stability and color fastness.

This requirement is satisfied by a fluorine resin, a polyolefin, e.g. a polypropylene, a high density polyethylene, an ultrahigh molecular weight polyethylene, a polyethylene terephthalate (PET), a polyethylene naphthalate (PEN) and a thermal plastic elastomer (TPE).

An Olefin TPE (TPO), a styrene TPE (SBC), a vinyl chloride TPE (TPVC), an urethane TPE (TPU), a polyester TPE (TPEE), a polyamide TPE (TPAE), a fluorine TPE (TPF), a polybutadiene TPE (RB), a polyisobutylene TPE, a silicone TPE and an ethylene- vinyl acetate TPE (EVA, EEA) have intermediate properties between rubbers and plastics.

According to the present invention, a styrene-ethylene-butadiene copolymer (SEBS), a styrene-butadiene copolymer (SBS), a styrene-isoprene copolymer (SIS), a styrene-isobutylene copolymer (SIBS) are preferably used in view of heat resistance property and elution property.

In addition to these TPEs, according to the present invention, a synthetic rubber, e.g. a regular butyl rubber, a halogenated butyl rubber, an isoprene rubber, a butadiene rubber, a styrene butadiene rubber, a nitrile rubber, a natural rubber, a rubber material containing EPDM, polybutadiene or polyisobutylene (including thermoplastic elastomer) as a major component and a kind of thermal plastic elastomer compound rubber.

At least a portion of a molded rubber article for sealing (made of a plastic material (e.g. a thermal plastic elastomer) or a rubber material) which may directly contact with stored chemicals needs to be made of an inactive and safest material.

For this material, a fluorine resin film is most suitable and a polytetrafluoroethylene (PTFE), a tetrafluoroethylene-perfluoro alkyl vinyl ether copolymer (PFA), a tetrafluoroethylene-ethylene copolymer (ETFE), a tetrafluoroethylene-hexafluoropropylene copolymer (FEP), a polychlorotrifluoroethylene (CTFE), a polyvinylidene fluoride (VdF), a trifluoroethylene-ethylene copolymer, a polytetrafluorodioxolecopolymer, a polyvinyl fluoride can be used as a fluorine resin film in view of a high light resistance property and a high vapor permeability, and PTFE, PFA, ETFE, FEP and VdF are preferable and PTFE is more preferable in view of a high antifouling property and a high heat resistance property.

A PTFE has an excellent chemical stability to prevent from dissolution and expansion in any chemicals and a good sliding property with a small mechanical friction coefficient.

A PTFE also has the highest melting temperature of 327°C among thermo-plastics and a melt flow rate (a general barometer which is indicative of a flow property of resins in a solution state above the melting temperature, and a PTFE has good characteristics of maintaining a uniform dispersed state above the melting temperature, keeping the shape after returning to the normal temperature and preventing from property degradation when it is formed in a film.

It is impossible to obtain desired adhesion strength even if a surface processing is applied to a fluorine resin film because the adhesion strength between a fluorine resin film and a sealing member is weak even when a fluorine resin film having such stable properties is used as a surface member of the sealing member.

Sufficient adhesion strength cannot be obtained for a certain kind of materials of the sealing member (rubber member) and the adhesion strength may become weak depending on a composition component in a rubber composition.

According to the present invention, a plastic layer having a good adherence property with a fluorine resin film and a bare rubber surface is interposed between them in order to improve the adhesion strength between the fluorine resin film and the bare rubber surface.

When a PTFE is chosen as a fluorine resin film, as an interposed film it is desirable to use a thermoplastic resin film (like as a PTFE film) having a property which is similar to a PTFE film under conditions of heating, cooling, irradiation of radial ray or electromagnetic ray, application of pressure and reduction in pressure and having a melt flow rate which is close to that of a PTFE.

A polyolefin film is suitable for such a material which satisfies the requirement of a good adherence property with both a fluorine resin film and a bare rubber surface. It is most suitable to use an ultrahigh molecular weight polyethylene with a molecular weight of 1 million to 7 millions, especially more than 5 millions.

It is preferable to choose a film having a property which is similar to that of a fluorine resin film when a fluorine resin film is not a PTFE. For example, such a film can be chosen from polypropylene, polyethylene, PET, PEN and nylon.

By forming a double film layer of a PTFE and an ultrahigh molecular weight polyethylene on the surface of a sealing molded rubber article which may directly contact with medicinal chemicals, it is possible to obtain a sufficient adhesion strength between the film layer and the bare rubber surface while avoiding an adverse effect to the medicinal chemicals, and thereby preventing detachment of a fluorine resin film layer from a bare rubber surface which has been a problem in the prior art.

According to the present invention, it is preferable to use a fluorine resin film of a thickness of 10-200 µm and an appropriate thickness can be determined in this range depending on application.

For example, the range of 30-200µm is preferable for a rubber plug for medical use. For actual production, if a range of 10-100µm is selected the void ratio of a thin film becomes low and the level of defectiveness becomes low.

A too thin fluorine resin film is not appropriate because production of the film becomes difficult and the laminating of a film on a molded rubber article becomes impossible by exceeding the handling limitation. A too thick fluorine resin film is also not appropriate because it may damage a sealing performance of a sealing molded rubber article according to the present invention.

It is preferable to use a polyolefin film of a thickness of 10-100µm. It is impossible to obtain a uniform adhesion strength because of a low accuracy in film thickness if a too thin polyolefin film is used. A too thick polyolefin film may damage the sealing performance of a sealing molded rubber article.

It is preferable to set the total thickness of a laminated double film layer of the fluorine resin film and the polyolefin film to 20-300µm.

A too thin double film layer is not practical because the film strength becomes too low. A too thick double film layer may damage the sealing performance of a sealing molded rubber article according to the present invention because the elasticity becomes too high.
A fluorine resin is inactive and does not form ion bonding nor covalent bonding with contacted surfaces. The hydrogen bonding formed by sharing hydrogen atoms on the surface where fluorine atoms are contacting is considered not so strong.

Although it is not clear where the origin of the high adhesion strength on the interface between the PTFE layer and the ultrahigh molecular weight polyethylene layer and the interface between the ultrahigh molecular weight polyethylene layer and the bare rubber surface comes from according to the present invention, it is considered to be based on a physicochemical theory of the bound interface, i.e. " the adhesion strength between two articles depends on a shape of the surface of the articles which contacts with an adhesive feature between two articles, and the adhesion strength is enhanced by the fact that a larger adhesive surface is obtained when the articles are wet".

According to the viscous elasticity characteristics and the understanding on a SP value (solubility parameter) which is used in a wettability theory, it is generally said that if the SP values of two materials are close the tensional force on the interface is small and the adhesion strength is high.
It is also said that if the SP values of two different materials are close the strength of the intermolecular forces becomes close, they come close each other and molecular from two materials is exchanged each other, and therefore two materials with close SP values can easily mix with each other, therefore they can be wet well and can be bound well.

A fluorine resin PTFE used in the present invention has the SP value (theoretical value) of 6.2 which is smallest among plastics.

Composite rubbers and natural rubbers have relatively small SP values in the range between 7 and 9. A polyethylene has a SP value of 7.7-8.4 which is about same or less than that of rubbers. It is considered that an ultrahigh molecular weight polyethylene has a SP value which is about same or less than that of rubbers but larger than that of PTFE.

An ultrahigh molecular weight polyethylene has a high stability because it has a high heat resistance and a high chemical resistance. It also has a melt flow rate of about 0 and a property which is close to that of PTFE.

It is considered that such a property contributes to the adherence property between an ultrahigh molecular weight polyethylene and a PTFE.

A fluorine resin film according to the present invention has a good adherence property even if no surface processing is applied.

It is possible to use a method for increasing a surface area per unit area by roughening the surface of a fluorine resin film in order to increase the adhesion strength.

As such a method, it is not appropriate to use a chemical processing method for using metallic sodium because the processed surface is discolored. A corona discharging or a sputter etching is more preferable because the processed surface is not discolored.

It is most preferable to use a sputter etching method because a corona discharging processing method requires large equipment for processing and the product control of processes films and unprocessed films becomes complicated.

A sealing molded rubber article having the aforementioned configuration according to the present invention can be realized by a process comprising putting a polyolefin film as an intermediate layer on a fluorine resin film, putting a sheet-shaped rubber member on the polyolefin film, molding the laminated films in a form of a molded rubber article by heating and applying pressure, thereby laminating of a fluorine resin layer with a high adhesion strength.

According to this process it is possible to obtain practically sufficient adhesion strength with a polyolefin film without applying the aforementioned various kinds of surface processing to the fluorine resin film, however a higher adhesion strength can be obtained by applying surface processing.

An appropriate size of fluorine resin film is put on a lower press mold tool having plural cavities, a polyolefin film is put on the fluorine resin film, a sheet-shaped rubber member is put on the polyolefin film (additionally a polyolefin film and a fluorine resin film are put on them if the both sides need to be laminated) and a upper press mold tool is moved down.

The lower press mold tool and the upper press mold tool are heated, a mold clamping pressure is applied to both the lower press mold tool and the upper press mold tool while vacuuming up the inside of the mold tools for deaerating the inside of the mold tools.

By this operation, the material of the sheet-shaped rubber member becomes fluid and enters in the cavities in a form of a laminated double film layer.

The sheet-shaped rubber member is compressed and heated in the cavities to cause diluting and cross-linking reaction. The molding is completed by removing the generated gas by vacuuming.

The heating temperature for the upper and lower mold tools is set to higher than the cross-linking temperature of the sheet-shaped rubber member and higher than the softening temperature of the rubber member if no cross-linking reaction is required. The mold clamping pressure is set to about 5 0 ∼ 200Kg / cm².

Sufficient diluting or cross-linking reaction cannot be realized below the required temperature and molding defects occur frequently because of insufficient diluting if the mold clamping pressure is lower than the required pressure.

The heating temperature for the upper and lower mold tools is properly set to a temperature (molding temperature) for the most appropriate viscosity for molding depending on the cross-linking temperature and softening characteristics of a rubber material.

Most of rubber materials can be molded with a mold clamping pressure of about 150 Kg / cm², and an appropriate mold clamping pressure is usually determined depending on the viscous elasticity of the rubber material.

After this step, an aimed molded rubber article is produced by opening the upper and lower mold tools, removing a molded article from the mold tools and cutting the surrounding part of the molded articles properly.

### (Embodiment 1)

A molded rubber article (a rubber plug) which has a cross section shown in Fig. 1(A) was produced by the following process.

Fig. 1(A) shows a rubber member 300 molded in a shape of a rubber plug, a fluorine resin film layer 100 for covering at least the surface of the rubber plug which may contact medicinal chemicals (the entire surface of the rubber plug in this example) and a polyolefin film layer 200 interposed between the rubber member 300 and the fluorine resin film layer 100.

A SIBS elastomer (Kaneka Corp. "SIBSTAR®") of 100 pts.wt. and a cross-linking agent (polysiloxane including hydroxy group) of 2 pts.wt. are kneaded by an open roll, and a sheet-shaped rubber member of a thickness of 10mm was obtained by heating after aging for 24 hours.

A PTFE film 100 of a thickness of 50µm and an ultrahigh molecular weight polyethylene film 200 of a thickness of 50µ m having a molecular weight of 5.5 millions were put on the lower mold tool LD of the mold tool shown in Fig.1(B) as a schematic cross sectional view. The sheet shaped rubber member 300 was put on the ultrahigh molecular weight polyethylene film 200, and additionally an ultrahigh molecular weight polyethylene film 200 and a PTFE film 100 were put on the sheet-shaped rubber member 300. The upper mold tool UD was moved down.

The upper and lower mold tools UD, LD were heated to 1 5 0 - 1 8 0 °C and the inside of the cavity CV was vacuumed up through the vacuuming outlet T. A molding under heating and pressurization was performed at a mold clamping pressure of 100 Kg / cm² for 10 minutes.

The molded article was cut along the cavity and a molded rubber article (a rubber plug) according to the present invention shown in Fig. 1(A) was obtained.

### (Comparative Example 1)

A PTFE film laminated rubber plug was obtained by a process which is same as that for Embodiment 1 other than that it used only PTFE films but used no ultrahigh molecular weight polyethylene film.

### (Adhesion strength test)

The following detachment (adhesion) strength test and reliability test were performed with respect to the rubber plugs which were obtained by Embodiment 1 and Comparative Example 1.

### (Detachment strength)

Reed-shaped test samples were made by laminating a PTFE film 100 on only one side of a sheet-shaped rubber member 300 with an interposed ultrahigh molecular weight polyethylene film 200 (Embodiment 1) and without an interposed ultrahigh molecular weight polyethylene film 200 (Comparative example 1) using processes which is similar to those for Embodiment 1 and Comparative example 1.

The end portion of the test samples were formed so that the sheet-shaped rubber member 300, the interposed ultrahigh molecular weight polyethylene film 200 and the PTFE film 100 were not adhered each other.

An end portion of the rubber member 300 of the test sample was fixed to a lower fixing jig and one end portion of the PTFE film 100 was fixed to an upper fixing jig. In case of Embodiment 1, one end portion of the ultrahigh molecular weight polyethylene film 200 was not fixed to the upper fixing jig, but only the PTFE film 100 was fixed to the upper fixing jig. The PTFE film 100 and the rubber member 300 were detached by pulling the upper fixing jig while moving up the upper fixing jig at a test speed of 500mm/min using an autograph (SHIMAZU Corp. Autograph AG-100B). A maximum loading value was measured at the time of detachment. The test was performed 10 times for each group of test samples.

A result of the measurement is shown in Table 1.

**[Table 1]**

| | Average Maximum Load (N) | Note |
|---|---|---|
| Embodiment 1 | 22.8 | Strong adhesion, fracture in rubber member |
| Comparative example 1 | 10.1 | Normal detachment |

As shown in Table 1, the PTFE film 100 was detached from the rubber member 300 at an average maximum load of about 10N in case of the test samples according to Comparative example 1. Detachment occurred neither at the interface between the PTFE film 100 and the ultrahigh molecular weight polyethylene film 200 nor the interface between the ultrahigh molecular weight polyethylene film 200 and the rubber member 300. The measurement ended by fracture in the rubber member 300 at the load of about 20N.

### (Reliability test)

### (Air sealing test)

Each of tow containers containing methyl alcohol of 160cc were sealed by a rubber plug according to Embodiment 1 and a rubber plug according to Comparative example 1 respectively, the two containers were put in an atmosphere of a temperature of 4 0 °C for 1 week, and the remaining volume of methyl alcohol in each container was measured. The measurement was performed 10 times for each case as the detachment strength test.

A result of the measurement is shown in Table 2.

**[Table 2]**

| | Average staying ratio (%) |
|---|---|
| Embodiment 1 | 99.7 |
| Comparative example 1 | 99.8 |

As shown in Table 2, no substantial decreased quantity was observed in Embodiment 1 and Comparative example 1. and no reduced sealing ability was observed.

## Claims

1. A molded rubber article for sealing a container or an instrument used in medication or medical care application, comprising a fluorine resin film layer (100) covering at least a surface of the molded rubber article which may contact with chemicals, **characterised in that** the molded rubber article further comprises a polyolefin film layer (200) being interposed between the fluorine resin film layer (100) and a bare rubber surface (300), wherein the polyolefin film layer (200) has a high adhesion property between the fluorine resin film layer (100) and the bare rubber surface (300) wherein the polyolefin is an ultrahigh molecular weight polyethylene with a molecular weight of 1 million to 7 millions and wherein the total thickness of the laminated double film layer of the fluorine resin film (100) and the ultrahigh molecular weight polyethylene film (200) is 20-300 µm.

2. A molded rubber article according to claim 1, wherein the rubber article is configured by a process comprising putting the ultrahigh molecular weight polyethylene film (200) as an intermediate layer between the fluorine resin film (100) and a sheet-shaped rubber member (300), and molding the stacked sheet/films in a form of a rubber plug by heating and applying pressure.

3. A molded rubber article according to claim 2, wherein the process further comprises using a double film of the ultrahigh molecular weight polyethylene film (200) and the fluorine resin film (100).

4. A molded rubber article according to Claim 1, wherein the rubber (300) comprises a thermoplastic elastomer.

5. A molded rubber article according to claim 4, wherein the thermoplastic elastomer is at least one selected from an olefin elastomer, a styrene elastomer, a vinyl chloride elastomer, an urethane elastomer, a polyester elastomer, a polyamide elastomer, a fluorine elastomer, a polybutadiene elastomer, a polyisobutylene elastomer, a silicone elastomer and an ethylene-vinyl acetate elastomer.

## Patentansprüche

1. Geformter Gummiartikel zum Versiegeln eines Behälters oder eines Instruments, der/das in der Medikation oder medizinischen Versorgung verwendet wird, umfassend eine Fluorharzfilmschicht (100), die zumindest eine Oberfläche des geformten Gummiartikels bedeckt, die mit Chemikalien in Kontakt kommen kann, **dadurch gekennzeichnet, dass** der geformte Gummiartikel ferner eine Polyolefinfilmschicht (200) umfasst, die zwischen der Fluorharzfilmschicht (100) und einer blanken Gummioberfläche (300) eingefügt ist, wobei die Polyolefinfilmschicht (200) eine hohe Adhäsionseigenschaft zwischen der Fluorharzfilmschicht (100) und der blanken Gummioberfläche (300) aufweist, wobei das Polyolefin ein ultrahochmolekulargewichtiges Polyethylen mit einem Molekulargewicht von 1 Million bis 7 Millionen ist, und wobei die Gesamtdicke der laminierten Doppelfilmschicht des Fluorharzfilms (100) und des ultrahochmolekulargewichtigen Polyethylenfilms (200) 20-300 µm beträgt.

2. Geformter Gummiartikel nach Anspruch 1, wobei der Gummiartikel durch ein Verfahren konfiguriert wird, bei dem der ultrahochmolekulargewichtige Polyethylenfilm (200) als eine Zwischenschicht zwischen den Fluorharzfilm (100) und ein blattförmiges Gummielement (300) eingebracht wird, und die gestapelten Blätter/Filme durch Erhitzen und Aufbringen von Druck in eine Form eines Gummistopfens geformt werden.

3. Geformter Gummiartikel nach Anspruch 2, wobei das Verfahren ferner die Verwendung eines Doppelfilms aus dem ultrahochmolekulargewichtigen Polyethylenfilm (200) und dem Fluorharzfilm (100) umfasst.

4. Geformter Gummiartikel nach Anspruch 1, wobei der Gummi (300) ein thermoplastisches Elastomer umfasst.

5. Geformter Gummiartikel nach Anspruch 4, wobei das thermoplastische Elastomer zumindest eines ausgewählt aus einem Olefinelastomer, einem Styrolelastomer, einem Vinylchloridelastomer, einem Urethanelastomer, einem Polyesterelastomer, einem Polyamidelastomer, einem Fluorelastomer, einem Polybutadienelastomer, einem Polyisobutylenelastomer, einem Silikonelastomer und einem Ethylen-Vinylacetatelastomer ist.

## Revendications

1. Article en caoutchouc moulé pour sceller un récipient ou un instrument utilisé dans une application de médication ou de soins médicaux, comprenant une couche de film de résine fluor (100) couvrant au moins une surface de l'article en caoutchouc moulé susceptible d'être en contact avec des produits chimiques, **caractérisé en ce que** l'article en caoutchouc moulé comprend en outre une couche de film polyoléfinique (200) interposée entre la couche de film de résine fluor (100) et une surface en caoutchouc nue (300), dans lequel la couche de film polyoléfinique (200) a une propriété d'adhérence élevée entre la couche de film de résine fluor (100) et la surface en caoutchouc nue (300) où la polyoléfine est un polyéthylène de poids moléculaire ultrahaut ayant un poids moléculaire de 1 à 7 millions et où l'épaisseur totale de la couche constituée du double film stratifié du film de résine fluor (100) et du film polyéthylène de poids moléculaire ultrahaut (200) est de 20 à 300 µm.

2. Article en caoutchouc moulé selon la revendication 1, dans lequel l'article en caoutchouc est conçu par un procédé comprenant le placement du film polyéthylène de poids moléculaire ultrahaut (200) à titre de couche intermédiaire entre le film de résine fluor (100) et un élément en caoutchouc en forme de feuille (300), et le moulage de l'empilement feuille/films sous la forme d'un bouchon en caoutchouc par chauffage et application de pression.

3. Article en caoutchouc moulé selon la revendication 2, dans lequel le procédé comprend en outre l'utilisation d'un double film constitué par le film polyéthylène de poids moléculaire ultrahaut (200) et le film de résine fluor (100).

4. Article en caoutchouc moulé selon la revendication 1, dans lequel le caoutchouc (300) comprend un élastomère thermoplastique.

5. Article en caoutchouc moulé selon la revendication 4, dans lequel l'élastomère thermoplastique est au moins un élastomère choisi parmi un élastomère oléfinique, un élastomère styrénique, un élastomère chlorure de vinyle, un élastomère uréthane, un élastomère polyester, un élastomère polyamide, un élastomère fluoré, un élastomère polybutadiène, un élastomère polyisobutylène, un élastomère silicone et un élastomère éthylène-acétate de vinyle.
